# EUROPEAN PATENT APPLICATION

(11) **EP 1 206 944 A1**
(43) Date of publication of application: **22.05.2002**
(21) Application number: 00128032.0
(22) Date of filing: 21.12.2000
(51) Int. Cl.: A61L 15/50, A61L 15/22

(54) **Improved apertured top sheet for absorbent articles**

(30) Priority: 06.11.2000 US 706934
(71) Applicant: Polymer Group, Inc., North Charleston, S.C. 29405 (US)
(72) Inventor: Kiyoshi, Inoshita, Sagamihara-Shi (JP); Dale, Robert, Lawenceville, GA 30043 (US); Karszes, William, Rosswell, GA 30076 (US)
(74) Representative: Flosdorff, Jürgen, Dr.

(57) **Abstract**

In the application and use of absorbent articles, prolonged contact with the material of the sheet, especially in the presence of moisture from exudates or excreta, have an adverse effect on the health of the users skin. It has been found that an improved top sheet material for use in the construction of absorbent articles may be constructed from thermoplastic polymers mixed with certain surface friction reducing agents. These surface friction reducing agents are selected from those used in the manufacture of cosmetic materials and are thus known to have no adverse human etiology. As the top sheet material is formed from thermally extruded films or fibers, it is requisite of the surface friction reducing agents that there is no thermal degradation that adversely effect the contact surface or fluidic transfer to the interior elements of an absorbent article.

## Description

### Cross Reference to Related Application

This application claims the benefits of provisional application Serial No. 60/164,032, filed November 5, 1999.

### Technical Field

The invention disclosed herein is directed to an apertured top sheet material for absorbent articles, such as diapers and sanitary devices, whereby the use of certain melt additives in the construction of the top sheet improves overall user comfort.

### Background of the Invention

The fabrication and application of absorbent articles are well known. Representative absorbent articles include the general structure of a liquid-retaining core centrally located between a liquid impervious back sheet and a liquid pervious top sheet. Core materials are selected from cellulosic pulp, superabsorbent particles or fibers, or from other liquid retaining materials. The back sheet must form a barrier to the external environment of the absorbent article and is usually selected from liquid impervious materials as may found in non-apertured films and nonwoven fabrics exhibiting significantly high hydraulic head values. The top sheet is typically selected from films or nonwoven fabrics constructed of thermoplastic polymers and having either inherent or induced apertures.

In the application of the absorbent article, the top sheet material performs the general function of separating the liquid laden absorbent core from the skin of the user. It is necessary that the top sheet maintain a relatively dry touch while allowing for the unhindered passage of liquid through into the core.

The absorbent article may be worn for an extended period, during which it has been found that contact between the top sheet and the user's skin result in adverse effects on the skin health. Representative adverse effects seen or complained of include erythema, contact dermatitis, and comedogenesis. It is postulated that the adverse effects caused by skin contact with the top sheet are induced by two general mechanisms, surface friction and prolonged exposure to moisture.

Surface friction results from the prolonged rubbing or abrading of the top sheet against the user's skin. It has been appreciated by those skilled in the art that an improvement in the tactile quality by reducing surface friction has been beneficial in improving user comfort. U.S. 6,120,783 to Roe, et al., U.S. 6,120,488 to VanRijswijck, et al., and U.S. 5,944,705 to Ducker, et al., employ a lotion composition or a plurality of lotion compositions that are applied topically to the top sheet material. This method for reducing surface friction, while exhibiting beneficial effects, requires a complex manufacturing process for the top sheet material while the top sheet is subject to decreased efficiency over the course of repeated liquid insult. U.S. 5,609,587 to Roe addresses the durability of the topical lotion treatment by incorporating yet additional chemistries to bind the lotion to the matrix, further complicating the manufacturing process.

Further complicating the selection of a suitable top sheet material is the effect of prolonged exposure to moisture. Moisture is present as human exudate or excreta, herein referred to in general and interchangeably as a fluid or liquid, that is to be absorbed and sequestered into the absorbent article. The most benign effect of moisture being present and in contact with the user's skin, though having the greatest impact on user acceptance, is the general discomfort induced by the sensation of being "wet". As exposure is maintained for longer periods or is recurrent, physiological effects result from the osmotic attack on the skin, leading to redness, itching, rash, and further complications including bacterial and fungal infection. U.S. 5,977,429 to Phillips, et al., address the problem by incorporating, below and into the top sheet, specialized profiled fibers that exhibit enhanced liquid retention. While the use of profiled fibers can expedite the transfer of fluid, a portion of the fluid insult remains in the intricacies of the top sheet.
It is desirable to develop an apertured, thermoplastic top sheet, which is more user-acceptable and yet retains the necessary functional properties of liquid transfer ultimately to the core element.

### Summary of the Invention

The present invention is directed to an apertured top sheet having reduced surface friction while maintaining dryness of touch and necessary liquid transfer properties over the service life of the absorbent article.

To obtain an apertured top sheet having said improved/maintained qualities that are essentially permanent, the immediate invention contemplates the incorporation of melt additives into a thermoplastic polymer prior to extrusion. The melt additives are selected from those having known performance in cosmetic compositions and having resistance to thermal degradation. Thermoplastic polymers that have been found to be the most advantageous include those selected from the family of polyolefins, including polypropylene, polyethylene, and copolymers thereof.

Melt additives exhibiting beneficial reduction in surface friction are selected from natural and synthetic materials utilized in the cosmetics industry having acceptable tactile smoothness and exhibiting no adverse etiology when applied to the skin directly. Said acceptable melt additives may be categorized as humectants or as lubricous powders, suitable grades being available from commercial cosmetic and chemical suppliers. Humectant materials that exhibit advantageous performance include natural oils and extracts as is typified by the cholestric and phytosteryl chemically modified macadamia nut oil and synthetic materials including derivatives of N-lauryl-L-glutamate, including di(cholestrin,oxidodecyl) N-lauryl-L-glutamate. Suitable lubricious powders including zinc complexes, including zinc oxide. It is to be understood that the melt additives may be used either singularly or in combination.

It is further contemplated that the resulting top sheet having said improved and maintained qualities can be formed by differing mechanisms. The homogenized polyolefin and cosmetic derived melt additive mixture may be extruded into a monolayer film or film having a plurality of layers, and being rendered apertured by methods known to those skilled in the art. In the alternative, said polymeric mixture can be extruded directly into fibers of continuous length. The continuous fibers may either be directly coalesced into a nonwoven fabric (i.e. by spunbond technology) or subsequently reduced to staple length and incorporated into a nonwoven fabric formed by carding and related technology.

It is further contemplated by the immediate invention that an apertured top sheet is constructed from an extruded admix of polyethylene and between 0.1% and 10% by weight of a suitable thermally stable cosmetic additive selected from humectants and lubricious powders, this top sheet being used to cover an absorbent core material, the construct being incorporated into an absorbent article.

Other features and advantages of the present invention will become readily apparent from the following detailed description, the accompanying drawings, and the appended claims.

### Brief Description of the Drawings

The invention will be more easily understood by a detailed explanation of the invention including drawings. Accordingly, drawings which are particularly suited for explaining the invention are attached herewith; however, is should be understood that such drawings are for explanation purposes only and are not necessarily to scale. The drawings are briefly described as follows:
Figure 1 is a cross sectional view of an absorbent sanitary article.
Figure 2 is a schematic representation of the processing apparatus for producing an apertured film top sheet in accordance with the principles of the present invention.

### Detailed Description

While the present invention is susceptible of embodiment in various forms, there is shown in the drawings and will hereinafter be described a presently preferred embodiment of the invention, with the understanding that the present disclosure is to be considered as an exemplification of the invention, and is not intended to limit the invention to the specific embodiment illustrated.

The formation of a material suitable as a top sheet can be accomplished by extruding the mix of polyolefin with the melt additive as either a film or a filament for subsequent processing into a nonwoven fabric.

A film can be formed by conventional methods in which the thermoplastic polymer, preferably polyolefin, is heated until the polymer has reached a molten state. The friction reducing melt additive, selected from the humectant or lubricous powder agents, is then admixed into the molten polymer in the preferable range of 0.1% to 10% by weight, 0.5% to 5.0% being most preferred. The selected friction reducing agents should not undergo deleterious thermal degradation at the melt and extrusion temperatures, on the order of 230°C. Other colorants and deblocking agents can be added to the mix. Upon the mixture reaching homogeneity, pressure is applied and the molten material is extruded through a slot die. Following extrusion, the newly formed film is apertured by any a number of methods, including but not limited to needlepunch, pin perforation, embossing and stretching, or roll forming.

Nonwoven fabrics can be produced directly by extruding the aforementioned admixed thermoplastic polymers and melt additives into continuous filaments or by reducing the continuous filament to staple length fibers and subsequently carding and integrating the staple fiber into a fabric. Methods for spinning thermoplastic polymers having incorporated melt additives into filament and fiber are well known in the art, and no unusual requirement is known or expected for attaining suitable product outside that skill set. A nonwoven fabric exhibits an inherent porosity as a result of the fibrous construction, however, further aperturing of the fabric can be conducted if deemed necessary

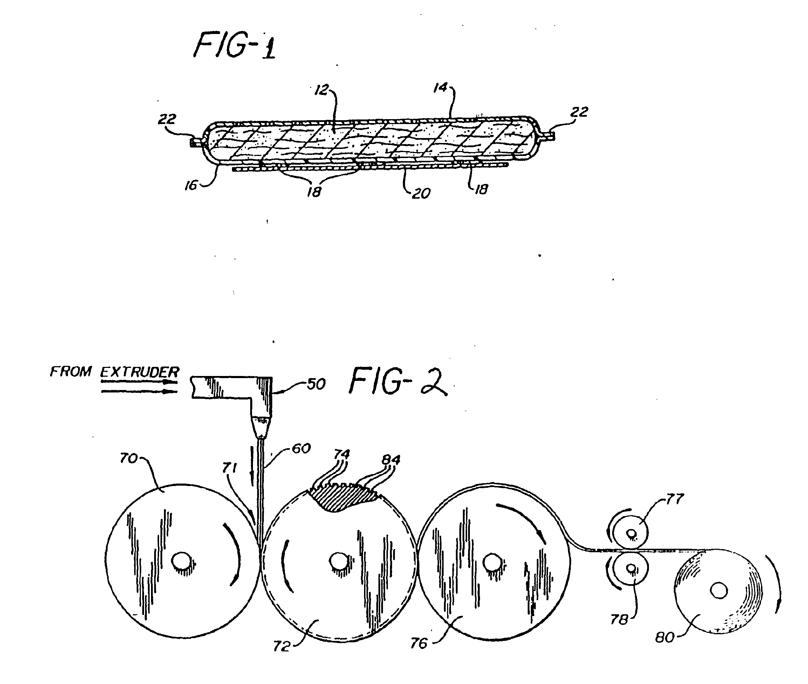

Figure 1 depicts an absorbent article in the form of a sanitary napkin as described in U.S. 4,690,679, incorporated herein by reference. This patent discloses a sanitary article having an apertured film as the top sheet. In this instance, a film is perforated after extrusion by passing the film through a set of nip rolls, one of said rolls carries a number of protuberances, thereby causing apertures to be formed. The film of reference has two layers, the top layer having a higher melting point than the bottom layer, thus affording easier heat sealing of the bottom layer (i.e. the backing sheet) to other components without undue fabrication complexity or deleterious effects on pliability.

In Figure 1, a sanitary napkin 10 is comprised of a central absorbent core 12, an apertured top sheet 14 in face to face juxtaposition with the absorbent core 12, and a liquid impervious film back sheet 16. The absorbent core 12 may be composed of any known materials exhibiting acceptable performance, such as cellulosic pulp or superabsorbent particles, the general construction being immaterial to the performance of the top sheet 14 of the present invention. Strips of pressure sensitive adhesive tape 18 may be provided on the outer surface of the sanitary napkin to enable attachment of the article to clothing. A release strip 20 may be applied over the adhesive strips 18 to prevent premature adhesion. The perimeter of the apertured top sheet 14 is heat sealed to the perimeter of the back sheet 16 as shown at 22.

A method of fabricating the inventive apertured top sheet as a film is included in said U.S. 4,690,679 patent. Therein, the top sheet is formed by co-extruding and aperturing a film comprising a layer of polyethylene juxtaposed onto a layer of ethylene vinyl acetate (hereinafter referred to EVA). The polyethylene layer of the film, which is in contact with the absorbent core, has a higher melting point than the EVA layer, and thus the EVA can be thermal bonded to form the absorbent article without deleterious effect of the skin contact side.

Referring to Fig. 2, there is schematically illustrated a process for preparing a two-layered, apertured film. Certain portions of the apparatus and processing steps used to produce the apertured film layer are disclosed generally in the prior art, e.g., in U.S. Patent No. 3,632,269 (Dovlak, et al.) and U.S. Patent No. 4,381,326 (Kelly).

The melt for the upper layer at a temperature of about 450 degrees F. and the melt for the lower layer at a temperature of about 440 degrees F. were then fed to a dual compartment, heated slot die 50 for coextrusion into a thin sheet. The slot die 50 contains a separator 52 which divides the interior of the die into separate compartments 54 and 56, as shown in Fig. 6. A temperature gradient is developed across the slot die, ranging from 415 degrees F. at the ends to 425 degrees F at the center. The LLDPE material was extruded through compartment 56, and the EVA material through compartment 54. The thick ness ratio of upper layer 42 and lower layer 44 was controlled by the weight outputs of the extruders as applied to the slot die to give a final product which comprised 35% by weight of the upper layer and 65% by weight of the lower layer.

From the slot die 50 the molten sheet 60 of coextruded materials drops directly into a nip 71 between a heated, smooth surfaced roll 70 and a resilient forming roll 72. Forming roll 72 had a resilient outer surface which was engraved with a pattern comprising a series of discontinuous elevated lands 74 separated by a continuous recessed area 84 as shown in exaggerated cross-section in Fig. 5. The shape of the apertures or holes in the apertured coextruded film corresponds generally to the shape of the land portions of the resilient forming roll 72 used in the coextrusion process. These land portions may take any desired geometrical shape such as rectangles, squares, hexagons, triangles, circles and the like. The size and number of lands on the forming roll should be selected to provide sufficient open area in the final apertured coextruded film to allow passage of body fluids, such as menstrual fluid, urine and the like, through the film into the absorbent core beneath. The lands were in the form of uniform hexagons, the sides of which measured 0.0104 inches. The hexagonal lands wer 0.0065 inches in height and had a center-to-center spacing of 0.028 inches. Roll 72 rotates at a speed from 1.5% to 8% faster than roll 70. As the rolls 70 and 72 rotate, they draw the coextruded material into nip 71, at which point the rolls cooperate in a wiping action to force substantially all of the coextrudate 60 into the continuous recessed areas 84 of the resilient forming roll 72. Rolls 70 and 72 are aligned to contact each other at an approximate presure of 65 lbs. per linear inch.

Heated roll 70 has a surface temperature of about 260 degrees F., whereas resilient forming roll 72 was internally cooled by 60 degrees F. water to provide an average surface temperature of about 200 degrees F. The resulting temperature differential between the two rolls, together with the engraved patterna on the forming roll, results in the sheet 60 being drawn around forming roll 72. The molten sheet begins to solidify in the desired form of an apertured film while it is in contact with the forming roll, the apertures of the film assuming the general shape of the lands 74 on the forming roll. It will be understood that the apertures in the film are formed by the action of the raised areas or lands 74 of the forming roll pressing through the molten coextrudate 60 just after it exits slot die 50.

The apertured film product passes from forming roll 72 to a chill roll 76. The chill roll 76 contacts the forming roll 72 at an approximate pressure of 55 lbs. per linear inch and is cooled to a temperature of about 65 degrees F. This brings the temperature of the apertured film to ambient conditions. The film is drawn off of the chill roll 76 by a pair of pull rolls 77 and 78, and the film is then rolled up on a take-up roll 80.

### Examples

### Example 1

A two-layer film top sheet was made according to U.S. 4,690,679, having a top or skin contact layer and a bottom or absorbent core contact layer. The top layer was composed of the following mixture (given in percentage by weight):

| | |
|---|---|
| Zinc oxide (surface friction reducing agent) | 1.00 |
| As available from Spartech-Polycom of Clayton, Missouri | |
| Titanium dioxide (opacifier) | 1.75 |
| As available from A. Schulman Inc. of Kerpen, Germany | |
| Linear Low Density Polyethylene | 97.25 |
| As available from Standard Oil Co. of San Francisco, California | |

The bottom layer was composed of the following mixture (again, given as percentage by weight):

| | |
|---|---|
| Titanium dioxide (opacifier) | 4.80 |
| As available from A. Schulman Inc. of Kerpen, Germany | |
| Atmer Concentrate (wetting agent) | 1.50 |
| As available from Spartech-Polycom of Calyton, Missouri | |
| Ethylene vinyl acetate | 93.70 |
| As available from Exxon Corp. of Irving, Texas | |

The two layers were independently melted and mixed using standard commercial extruders. Upon reaching homogeneity, the mixtures were directed though separate filter packs and melt pumps to a combining block, or alternately, a duel chamber die. At the combining block, the top layer was proportioned to approximately 35% total film weight and the bottom layer was proportioned to approximately 65% total film weight. The now interposed layers were spread into a uniform thickness by the application of a coat hanger die at approximately 700 pounds per hour. Subsequently, the film passed through a pressure nip wherein a cooled pattern engraved rubber roll rotates between 0.1% to 7% total rotations per minutes faster than an opposed heated Teflon.RTM coated roll, thus inducing apertures into the film. Typical open area of the apertured film range between approximately 20% and 50%, dependent upon the pattern used on the engraved rubber roll. The now apertured film was stripped from the cooled pattern engraved roll by a cooled smooth roll at approximately 255 feet per minute. At this point, the film was imparted with an optional embossed aesthetic pattern. The finished two-layer film top sheet exhibited a nominal thickness of 0.25 millimeter.

### Examples 2 through 7

Comparative two-layer film materials were fabricated by the means above. The top layer was composed in the alternative with the surface friction reducing melt additives listed in the chart below, the remainder of the volume being adjusted with the amount of linear low density polyethylene added.

| Example | Melt Additive | Addition by Weight | Permeability | Surface Friction |
|---|---|---|---|---|
| 2 | None | 0 | Control | Control |
| 3 | Macadamia nut oil - phytosteryl derivative | 0.5% | Same as Control | Reduced Friction |
| 4 | Zinc oxide | 1.0% | Same as Control | Reduced Friction |
| 5 | Silicone Oil | 1.0% | Decreased Permeability | Increased Friction |
| 6 | Silicone Oil w/ Macadamia nut oil - phytosteryl derivative | 1.0% 0.25% | Decreased Permeability | Increased Friction |

As may be seen from the above chart, humectants in the form of dervatized macadamia nut oil and lubricous powder agents in the form of zinc oxide provide an improved tactile performance without deleteriously effecting the permeability. Comparative application of silicone oil was not found to give beneficial performance and compromised permeability.

From the foregoing, it will be observed that numerous modifications and variations can be affected without departing from the true spirit and scope of the novel concept of the present invention. It is to be understood that no limitation with respect to the specific embodiments illustrated herein is intended or should be inferred. The disclosure is intended to cover, by the appended claims, all such modifications as fall within the scope of the claims.

## Claims

1. A top sheet for an absorbent article, comprising of;
a thermoplastic polymer having incorporated therein a surface friction reducing melt additive, said thermoplastic polymer being formed into a liquid pervious construction having apertures, said surface friction reducing melt additive performing the function of reducing the contact friction experienced between the top sheet and the skin of the user,

2. An apertured top sheet as in Claim 1, wherein the thermoplastic polymer is a polyolefin.

3. An apertured top sheet as in Claim 2, wherein the thermoplastic polymer is polyethylene.

4. An apertured top sheet as in Claim 1, wherein said surface friction reducing melt additive is selected from the group consisting of zinc oxide, derivitized macadamia nut oil, and combinations thereof.

5. An apertured top sheet as in Claim 1, wherein the blend ratio of surface friction reducing melt additive is present in the total amount of from about 0.1 to 10.0 percent by weight thermoplastic polymer.

6. An apertured top sheet as in Claim 5, where in the blend ratio of surface friction reducing melt additive is present in the total amount of from about 0.5 to 5.0 percent by weight thermoplastic polymer.

7. An apertured top sheet as in Claim 1, wherein the liquid pervious structure is a nonwoven fabric.

8. An apertured top sheet as in Claim 1, wherein the liquid pervious structure is a film.

9. An apertured top sheet as in Claim 8, wherein the film is comprised of a plurality of thermoplastic polymer and melt additive layers.

10. An apertured top sheet as in Claim 9, wherein the thermoplastic polymer of at least one thermoplastic polymer and melt additive layer is comprised of ethylene vinyl acetate.

11. An apertured top sheet as in Claim 9, wherein the thermoplastic polymer and melt additive layers have differing blend ratios.

12. An apertured top sheet as in Claim 9, wherein the thermoplastic polymer and melt additive layers have differing surface friction reducing melt additives.

13. An apertured top sheet as in Claim 8, wherein the film has apertures induced by the application of a nip roll.

14. A top sheet for an absorbent article, comprising of;
an apertured film of 95.0% to 99.5% by weight thermoplastic polymer, the thermoplastic polymer being polyethylene, and 5% to 0.5% by weight surface friction reducing melt additive, the surface friction reducing melt additive being zinc oxide, the total open surface area of the apertured surface being between 20% and 50%.

15. A top sheet for an absorbent article comprising of;
a skin contact layer,
an absorbent core contact layer,
the skin contact layer being composed of 95.0% to 99.5% by weight thermoplastic polymer, the thermoplastic polymer being polyethylene, and 5% to 0.5% by weight surface friction reducing melt additive, the surface friction reducing melt additive being zinc oxide,
the absorbent core contact layer being composed of 95.0% to 99.5% by weight thermoplastic polymer, the thermoplastic polymer being ethylene vinyl acetate,
the skin contact layer and the absorbent core contact layer being coextruded into a single film,
the coextruded film being rendered apertured by application of a nip roll, and
the total open surface area of the apertured coextruded film being between 20% and 50%.

16. An apertured top sheet as in Claim 15, wherein the skin contact layer comprises 35% of the total film weight.
